# EUROPEAN PATENT SPECIFICATION

(11) **EP 0 831 864 B1**
(45) Date of publication and mention of the grant of the patent: **12.09.2001**
(21) Application number: 96917725.2
(22) Date of filing: 05.06.1996
(51) Int. Cl.: A61K 35/78

(54) **PROCESSED GINSENG HAVING ENHANCED PHARMACOLOGICAL EFFECT**
BEARBEITETER GINSENG MIT VERSTÄRKTEM PHARMAKOLOGISCHEN EFFEKT
GINSENG TRANSFORME PRESENTANT UN EFFET PHARMACOLOGIQUE AMELIORE

(30) Priority: 07.06.1995 KR 1497395
(43) Date of publication of application: 01.04.1998
(73) Proprietor: Cheil Je Dang Co., Seoul 100-095 (KR); Park, Man, Ki, Seoul 135-010 (KR)
(72) Inventor: PARK, Man, Ki, Seoul 135-010 (KR); KIM, Nak, Doo, Shinbanpo 7th Apartment 302-304, Seoul 137-030 (KR); LEE, Seung, Ki, Hanshin Hankang Apartment 5-803, Seoul 137-040 (KR); PARK, Jeong, H., Hansol Village Apartment 301-208, Seoul 135-230 (KR); KIM, Jong, Moon, Chukong 2nd Apartment 229-313, Seoul 138-222 (KR)
(74) Representative: Wablat, Wolfgang, Dr.Dr.
(86) International application number: KR9600087
(87) International publication number: WO9640181

(56) References cited:
- FR-A- 2 671 488
- FR-A- 2 712 191
- PATENT ABSTRACTS OF JAPAN, Vol. 6, No. 223, (C-133), 1982; & JP,A,57 128 632 (HIROMU).
- PATENT ABSTRACTS OF JAPAN, Vol. 17, No. 270, (C-1063), 1993; & JP,A,05 009 123 (ISAO).

## Description

### TECHNICAL FIELD

The present invention relates to a processed ginseng having an enhanced pharmacological effect and a beverage composition comprising the same. More specifically, the present invention relates to a processed ginseng produced by heating ginseng at a high temperature, of which the ratio of ginsenoside (Rg₃+Rg₅)/(Rc+Rd+Rb₁+Rb₂) is above 1.0 and thus exhibits an enhanced pharmacological activity, and to a beverage composition comprising said processed ginseng.

### BACKGROUND ART

Since ancient times, ginseng has been widely known as the most typical nutritive and tonics. Recently, many scientific results on the components and pharmacological effects of ginseng have been reported, thereby the mysterious pharmacological effects of ginseng have been come to light under modern science. Hitherto, various pharmacological effects of ginseng such as suppression of aging, anti-arteriosclerosis, improvement of hyperlipidemia, improvement of liver function, defense against radiation hazard, immunological enhancement, anti-thrombotic effect, improvement of cerebral function, anti-stress, decrease of blood sugar, decrease of blood pressure, anti-tumor effect, etc. have been known.

Ginseng has been generally used in the form of fresh ginseng as it is harvested after cultivation, white ginseng produced by drying the fresh ginseng at normal temperature, or red ginseng prepared by heating the fresh ginseng at 98 to 100°C.

Particularly, the red ginseng is recognized as a very valuable herbal drug because its pharmacological effect is more potent than that of white ginseng. Recently, many researches on the minor components in red ginseng have been actively conducted, and as a result, an interest in novel pharmacological effects of those minor components has greatly increased. Particularly, since the minor components are produced during the process for preparing red ginseng by heating the fresh ginseng, they are appreciated as the specific components which can explain the excellent effect of red ginseng. The specific components identified as contained in the red ginseng only until now are saponins such as ginsenoside Rh2, Rs1, Rs2, Rg3, Rg5, Rh1, etc., polyacetylene compounds such as panaxytriol, etc. and the like. Such components are produced during the procedures for preparing the red ginseng and are present in a very minor amounts in the red ginseng.

The red ginseng is generally prepared by steaming the fresh ginseng for about 2 hours at a temperature of 98 to 100°C. The quality of red ginseng is mainly estimated on the basis of the apparent shape. Specifically, the red ginseng which has a good shape with no inside pores, cracks, inside whites, white peels, etc. is classified as a high-grade one. Accordingly, in order to produce a red ginseng having a better apparent shape there has been developed the method for preparing red ginseng by carrying out the heating procedure at the temperature of less than 98°C as low as possible. Said method is the subject of the pending patent application(Korean Patent Publication No. 92-5995). Since the apparent shape of a ginseng is regarded as the main standard for judgement of the commercial value thereof, as aforementioned, it was not attempted to heat the ginseng at a high temperature of 100°C or more.

However, recently some attempts to treat the ginseng at a high temperature have been made. For example, Japanese Laid-Open Patent Publication No. (Sho) 62-158,490 discloses a method for preparing tissue cultures having an increased content of ginsenoside Rh by heating the tissue cultures of ginseng at 110 to 160°C. However, since this method processes the tissue cultures of ginseng, not the ginseng itself, the processed product does not have the shape of ginseng, and further the tissue cultures of ginseng has different components and composition thereof as compared with the original ginseng cultivated in nature (see: Korean Journal of Pharmacognosy, 16, 171-171, 1985) and thus, natually has the phamacological effect different from that of original ginseng. Moreover, this method is complicated and uneconomical since it must be carried out through tissue culture step. In addition, some other methods for heating ginseng at a high temperature were attempted. However, they are merely the step utilized in the procedure for preparing cosmetics or teas, and the pharmacological effect of ginseng resulted from such high temperature-treatment has not even been studied.

### DISCLOSURE OF INVENTION

Thus, the present inventors have scientifically studied about the components and pharmacological effects, particularly processing methods and physiological activities of the ginseng. As a result of such studies, we have identified that when ginseng such as fresh ginseng, white ginseng, fine root of ginseng or an extract thereof treated at a much higher temperature than the temperature generally used for preparation of red ginseng the components which are contained merely in minor amounts in red ginseng greatly increase and new components which are not contained in red ginseng are formed, and therefore, the pharmacological activities of ginseng are highly enhanced. Thus, we have completed the present invention.

Therefore, it is an object of the present invention to provide a processed ginseng having enhanced pharmacological effect which is prepared by heating a ginseng at a high temperature.

More specifically, the present invention relates to a processed ginseng prepared by heating a ginseng for 0.5 to 20 hours at a high temperature of 120 to 180°C wherein the sum of ginsenosides Rg₃ and Rg₅ is always greater than the sum of ginsenosides Rc, Rd, Rb₁ and Rb₂, that is the ratio of ginsenoside (Rg₃+Rg₅)/(Rc+Rd+Rb₁+Rb₂) is above 1.0.

It is a further object of the present invention to provide a beverage composition comprising the processed ginseng as mentioned above.

### BRIEF DESCRIPTION OF DRAWINGS

For a thorough understanding of the nature and objects of the invention, reference should be made to the following detailed description taken in connection with the accompanying drawings in which :
Figure 1 represents the change in an amount of the essential oil fraction in accordance with the change of heating conditions(temperature and time) for fresh ginseng;
Figure 2 represents gas chromatograms of the essential oil fractions of the processed ginsengs obtained by heating ginseng for 2 hours at 120°C (Figure 2-b); and for 2 hours at 150°C (Figure 2-c) according to the present invention in comparison with gas chromatogram of the essential oil fraction of fresh ginseng which is not heat-treated (Figure 2-a);
Figure 3 represents an anti-oxidant activity of the processed ginsengs prepared by heating ginseng for 2 hours (-●-), 3 hours (-■-) at 120°C and for 2 hours at 110°C (-▲-) in comparison with that of white ginseng not heated (-◆-) and red ginseng commercially available(---); and
Figure 4 represents a vasodilation activity of the processed ginseng of the present invention prepared by heating ginseng for 2 hours at 120°C (-▲-) in comparison with that of ginseng treated at 110°C for 2 hours (-●-) , red ginseng (-O-) and white ginseng (-Δ-).

### BEST MODE FOR CARRYING OUT THE INVENTION

According to the present invention, a processed ginseng having an enhanced pharmacological effect is obtained by heating a ginseng at a high temperature to increase the content of the pharmacologically active components. The kind of ginseng which can be heated according to the present invention is not specially restricted and any of fresh ginseng, white ginseng, fine root of ginseng or leaves of ginseng plants or extracts thereof can be used in an intact form or a finely divided or a powder. Although the ginseng to be treated according to the present invention is generally *Panax ginseng, Panax quinquefolium* or *Panax notoginseng* can also be used in the present invention.

In the present invention, the heat-treatment of ginseng is carried out for 0.5 to 20 hours at 120 to 180°C, preferably for 2 to 5 hours at 120 to 140°C. The time required for heating depends on the heat temperature. Specifically, the heat-treatment should be conducted for a long time at a low temperature but can be conducted for a relatively short time at a high temperature. The heating is carried out utilizing hot air, steam, nitrogen, helium, carbon dioxide or mixed gas thereof. It is preferable to carry out the heating in a sealed container such as an autoclave to increase the heating efficiency. If desired, it may be preferable to introduce some water into the container or to heat the ginseng immersed in water in the sealed container.

If necessary, the processed ginseng thus prepared can be dried according to a known method at a lower temperature than the temperature at the preceding heating step, for example at the normal temperature to 80°C, to obtain the dried processed ginseng or a powder thereof.

In addition, if desired, the processed ginseng can be extracted according to a known method to prepare the processed ginseng extract. Specifically, the processed ginseng can be extracted with water, lower alcohol (for example, methanol, ethanol, etc.), lower ketone (for example, acetone, methylethylketone, etc.), super critical fluid or a mixed solvent thereof, and then the solvent is removed from the extract by concentration or lyophilization to prepare the processed ginseng extract in the form of a dried powder.

According to the present invention, similar results can be obtained by treating leaves of ginseng plants, instead of the ginseng itself, according to the same manner. Until now, the leaves of ginseng plants are not used for medicinal purposes but has generally been wasted or used merely as feedstuffs. Only some of them have been used as a source of cosmetics or food. But, when the leaves of ginseng plants are heated according to the process of the present invention, the pharmacological effect thereof much more increases, and therefore they can be used even for medicinal purposes as well as for their original use known heretofore.

Furthermore, according to the present invention the ginseng extract or the extract fraction can be subjected to heat-treatment according to the same manner as applied for fresh ginseng, white ginseng or fine root of ginseng to obtain the processed ginseng extract. In this case, the use of the ginseng extract can provide the merits that since the sample volume is reduced and therefore the device for processing ginseng can have a smaller size, the energy required for heating can be saved and the heating conditions including time and temperature can be controlled more precisely.

The processed ginseng of the present invention prepared according to the method as mentioned above shows a greatly enhanced pharmacological effect because it contains several components, for example nonpolar saponins, phenolic compounds, polyacetylene compounds, etc. in large quantities, which are contained merely in infinitesimal quantities or not contained in fresh ginseng, white ginseng or red ginseng. That is, in the processed ginseng the various new volatile components produced during the heating step are present and they have been identified as having anti-oxidant activity, anti-tumor activity, and the like. The processed ginseng of the present invention contains various saponins, for example, ginsenosides Rg₃, Rg₅, Rh₁, Rh₂, Rh₃, Rh₄, F₄, etc. which are not contained or contained merely in infinitesimal quantities in the white ginseng or red ginseng. Among the saponins, ginsenosides F₄, Rg₃, Rg₅ (Δ 20-ginsenoside Rg₃) are contained particularly in large quantities. Particularly, the processed ginseng according to the present invention exhibits valuable physiological activities since the ratio of (Rg₃+Rg₅)/(Rc+Rd+Rb₁+Rb₂) is above 1.0 in contrast to the fresh ginseng or red ginseng in which ginsenoside components such as Rg₃ and Rg₅ are substantially not present.

The processed ginseng having an enhanced pharmacological effect prepared by heating ginseng according to the present invention, for example, processed ginseng powder, processed ginseng extract, etc., can be used as a source having a more potent efficacy in the field of all kinds of pharmaceuticals, herb medicines, health food, food, teas, cosmetics and the like, in which the ginsengs have been conventionally used. More particularly, the processed ginseng of the present invention can be prepared in the form of a beverage composition.

Accordingly, the present invention also provides a beverage composition which comprises the processed ginseng in the form of a powder or an extract. It is appropriate for the beverage composition according to the present invention to contain the dried processed ginseng in an amount of 200 to 2000mg, preferably 400 to 1000mg, for a single dosage.

If necessary, the beverage composition of the present invention can additionally contain the extracts of medicinal herbs such as Zingiberis rhizoma, Ziziphi fructus, Cinnamomi cortex, Lycii fructus, Polygalae radix, Astragali radix, etc. The additives such as sweeteners, flavors, preservatives and the like which are conventionally used in a beverage composition can also be contained in the beverage composition of the present invention.

The present invention is more specifically explained by the following examples, test examples and composition examples. However, it should be understood that the present invention is not limited to those examples in any manner.

### EXAMPLE 1

1kg of fine root of ginseng was introduced into an autoclave and then heated with steam for 3 hours at 120°C. The heat-treated ginseng was removed from the autoclave and dried at temperature of 50 to 60°C to obtain the desired processed ginseng.

### EXAMPLE 2

1kg of fresh ginseng(main root) was introduced into an autoclave and then heated with steam for 2 hours at 130°C. The heat-treated ginseng was removed from the autoclave and dried at temperature of 50 to 60°C to obtain the desired processed ginseng.

### EXAMPLE 3

1kg of dried fine root of ginseng was extracted by refluxing with 2L of methanol for 4 hours under water bath and then filtered. The ginseng extract thus obtained was dried under reduced pressure. The resulting ginseng extract in the form of syrup was introduced into an autoclave and then heated for 4 hours at 120°C. Then, the heat-treated ginseng extract was dried at 60°C to prepare the processed ginseng extract in the form of a concentrated syrup.

### EXAMPLE 4

The processed ginseng prepared in Example 1 was extracted by refluxing with methanol for 4 hours under water bath and then filtered. The ginseng extract thus obtained was dried under reduced pressure to obtain the processed ginseng extract in the form of a powder.

### TEST EXAMPLE 1

### Analysis of saponins contained in the processed ginseng of the present invention

The saponin components contained in each of the processed ginseng according to the present invention, fresh ginseng, white ginseng and red ginseng were analyzed by the method described in the following.

5g of white ginseng and 5ml of water were introduced into each of four 40ml stainless steel containers which were then sealed. The mixtures were heated for 2 hours at 110°C, for 2 hours at 120°C, for 3 hours at 120°C and for 2 hours at 130°C, respectively. 5g each of the heat-treated processed ginseng obtained above, commercially available white ginseng and red ginseng was extracted 3 times with 100ml of methanol and then the extract was concentrated. The residue was suspended in water and extracted 3 times with 100ml of ether. The remaining aqueous layer was extracted 3 times with 100ml of butanol and then the butanol fraction was concentrated. The concentrate was dissolved in methanol and analyzed by HPLC(column: LiChrosorb NH₂, mobile phase: CH₃CN/H₂O/i-PrOH=80/5/15→80/20/15, detector: ELSD(Evaporative light scattering detector)). The measured results are described in the following Table 1.

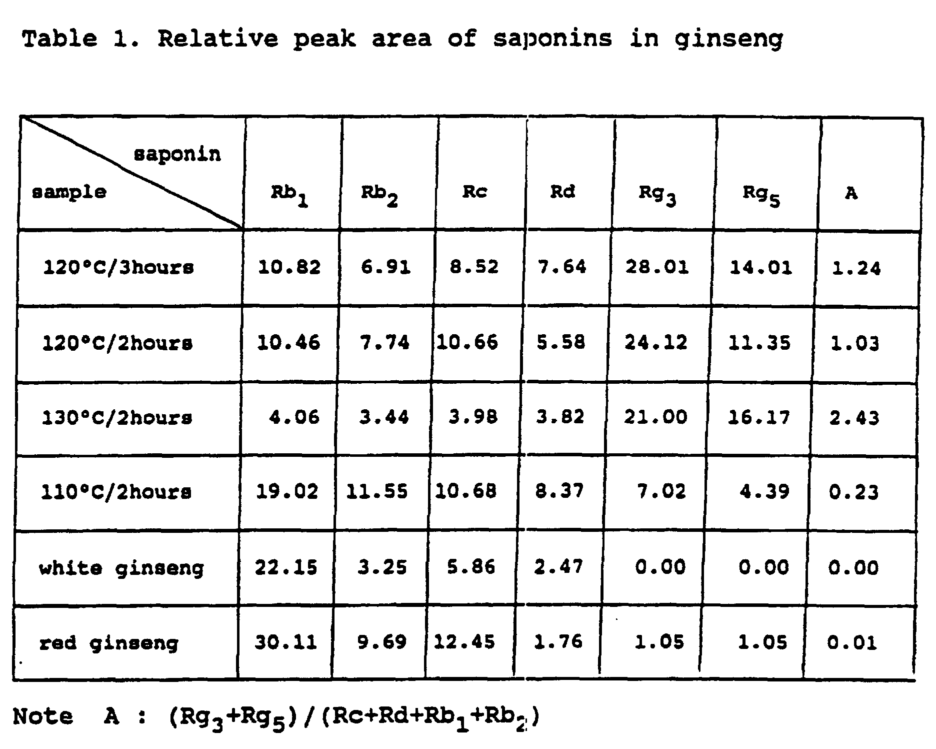

From the results described in Table 1 above, it can be seen that the processed ginseng obtained by heating ginseng according to the present invention can show an excellent pharmacological effect due to a remarkable increase in content of ginseng saponins Rg₃ and Rg₅ which are not or substantially not present in fresh ginseng, white ginseng and red ginseng.

On the basis of the results as mentioned above, in order to identify more specifically the content change of saponins, particularly ginsenosides Rg₃ and Rg₅ in accordance with the heating temperature change, the contents of Rg₃ and Rg₅ in the ginsengs heated for 2 hours at 100°C, 110°C, 120°C, 130°C, 150°C, 160°C, 180°C and 200°C, respectively, were determined and compared with the ginseng which is not heated(fresh ginseng). The results are described in the following Table 2.

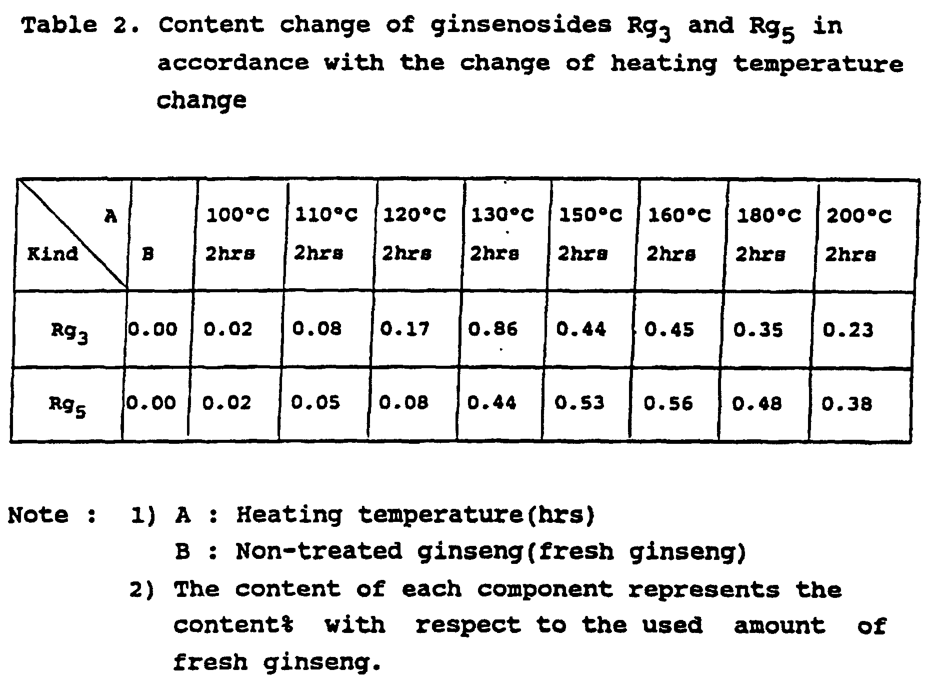

It can be seen from the above results that the contents of ginsenosides such as Rg₃ and Rg₅ in the processed ginseng heated at 120 to 180°C according to the present invention have greatly increased in contrast to the non-treated fresh ginseng or red ginseng(heated at 100°C). In case the heating is carried out at a high temperature of 180°C or more, although the contents of ginsenosides Rg₃ and Rg₅ in the processed ginseng are somewhat higher than in the fresh ginseng or red ginseng, such a high temperature-heating is not desirable because special equipments or excessive costs are needed for heating at such a high temperature.

### TEST EXAMPLE 2

### Amount of essential oil fraction according to the heating conditions

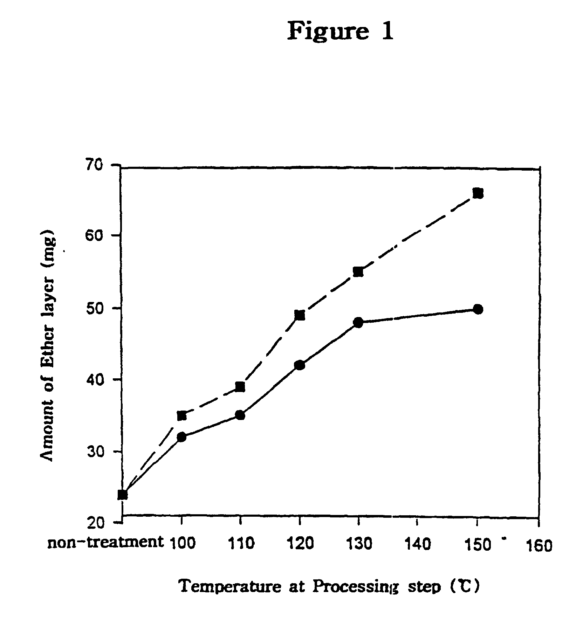

10 containers were divided into 2 groups with 5 per each group. The first group will be heated for 2 hours and the second group will be heated for 4 hours, at 100°C, 110°C, 120°C, 130°C and 150°C, respectively. 10g of fresh ginseng was introduced into each containers and heated for 2 or 4 hours at 100°C, 110°C, 120°C, 130°C and 150°C, respectively, and then extracted with 200ml of methanol under refluxing. Methanol was removed from the extract by evaporation to obtain the methanol extract. This extract was suspended in 100ml of water and then extracted 3 times with 100ml of ether. The extracted ether fractions were combined and the solvent was removed by evaporation. The weight of the remaining essential oil fraction was measured to observe the change in amount of essential oil fraction according to the change of heating temperature and time. The measured results are depicted in Figure 1.

It has been reported that the essential oil fraction of ginseng contains phenolic components, polyacetylene compounds, etc. which have anti-oxidant effect, anti-tumor effect and the like. From the result illustrated in Figure 1, it can be seen that the amount of essential oil fraction contained in the processed ginseng of the present invention greatly increases in contrast to fresh ginseng which is not heated or red ginseng heated at 100°C. This means that the processed ginseng of the present invention can show much more potent anti-oxidant and anti-tumor effects than the fresh ginseng or red ginseng.

### TEST EXAMPLE 3

### Gas chromatogram of essential oil fraction of the processed ginseng

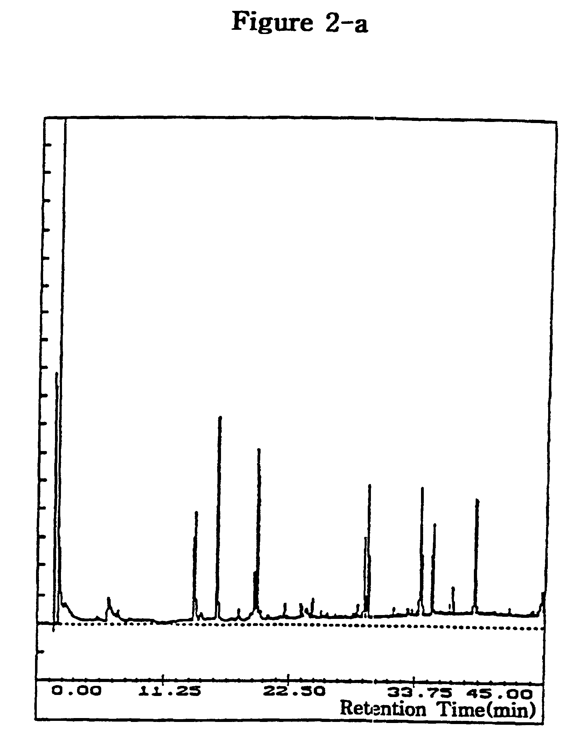

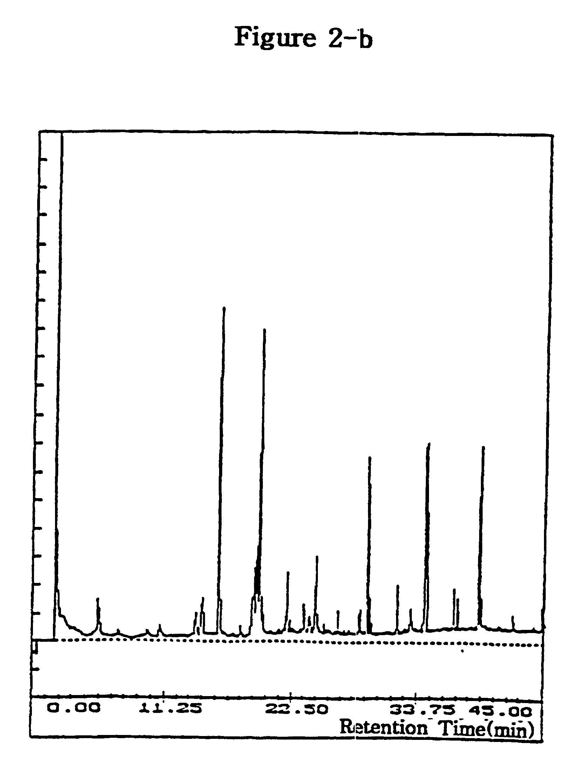

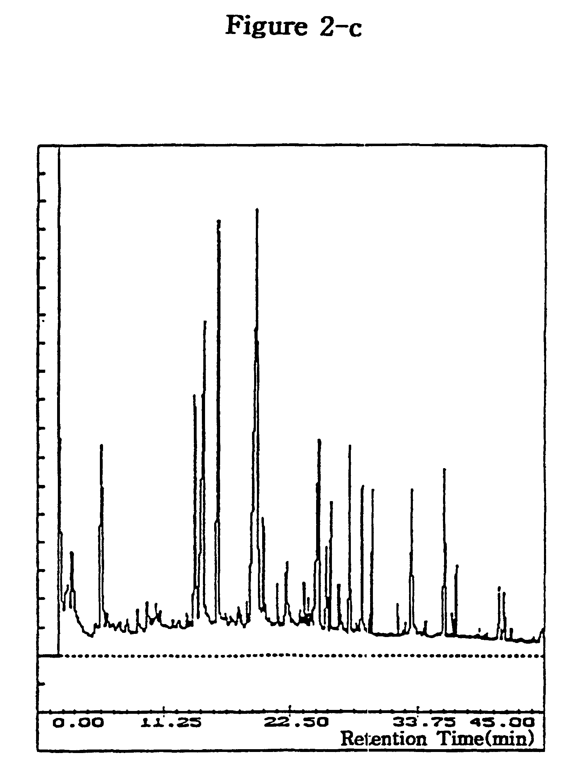

10g of fresh ginseng was introduced into each of two sealed containers and heated for 2 hours at 120°C and 150°C, respectively. After heating, the fresh ginseng was extracted with 200ml of methanol under refluxing and then the methanol was removed by evaporation. The residue was suspended in 100ml of water and then extracted with 100ml of ether. The ether fraction was concentrated to obtain the essential oil fraction which was dissolved in 2ml of chloroform and then subjected to gas chromatography (conditions: column OV-1 25m, capillary column, increasing the oven temperature from 170°C to 280°C, detector FID) to examine the components of the essential oil fraction. In addition, the essential oil fraction extracted from the fresh ginseng which is not heat-treated was examined according to the same method as mentioned above. The obtained results are depicted in Figure 2.

The essential oil fraction of ginseng contains nonpolar compounds such as phenolic compounds, polyacetylene compounds, etc., which have been known as having the anti-oxidant effect, anti-tumor effect, and the like. Figure 2-a is the gas chromatogram for ginseng not processed, Figure 2-b is the gas chromatogram for ginseng heated for 2 hours at 120°C and Figure 2-c is the gas chromatogram for ginseng heated for 2 hours at 150°C. From these gas chromatograms, it can be identified that the content of nonpolar components such as volatile essential oil components, polyacetylene compounds, and the like increases and new components are produced according to the increase of heating temperature. This result means that the anti-oxidant effect of ginseng increases according as the heating temperature is raised.

### TEST EXAMPLE 4

### Anti-oxidant effect of the processed ginseng

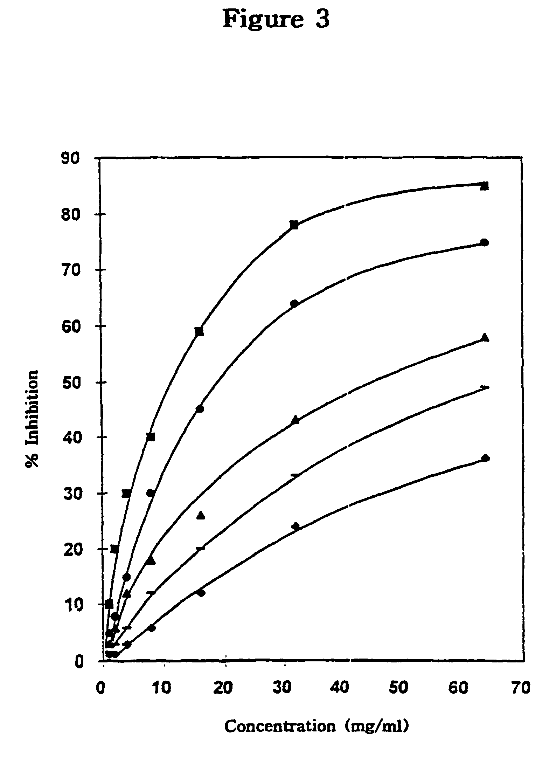

5g of white ginseng and 5ml of water were introduced into each of three 40ml stainless steel containers which were then sealed and heated for 2 hours at 110°C, for 2 hours at 120°C and for 3 hours at 120°C, respectively. The heat-treated processed ginseng was extracted with 200ml of methanol under refluxing and then filtered. Methanol was removed from the filtrate by evaporation under reduced pressure and the residue was suspended in 100ml of water and then extracted 3 times with 100ml of butanol saturated with water. The butanol fraction was concentrated under reduced pressure to remove butanol and the residue was dissolved in 20ml of ethanol. This solution was diluted gradually with ethanol to prepare 1/2, 1/4, 1/8, 1/16 and 1/32 dilution samples. In addition, white ginseng and red ginseng were extracted and diluted to prepare sample solutions according to the same procedure as mentioned above except the heating step is excluded. 0.1ml of each of the sample solutions was introduced into a test tube and 1.9ml of 0.004% DPPH(diphenylpicrylhydrazyl)-ethanol solution was added thereto. The resulting solution was heated for 30 minutes at 37°C and then the absorbance at 515nm was measured. The measured results are depicted in Figure 3.

From the results illustrated in Figure 3, it can be seen that the extract of ginseng processed at a high temperature (120°C) according to the present invention exhibits a remarkably improved anti-oxidant activity compared with the unprocessed ginseng. Such an anti-oxidant activity increases dependently on the dose. Therefore, in view of 50% inhibition concentration, the anti-oxidant effect of the ginseng processed for 2 hours at 120°C is about 4 times greater than that of the ginseng processed for 2 hours at 110°C as well as red ginseng. From the results above, it can be seen that the processed ginseng prepared according to the present invention in which the ratio of (Rg₃+Rg₅)/(Rc+Rd+Rb₁+Rb₂) is above 1.0 has more potent nutritive and tonic effect in comparison with fresh ginseng or red ginseng.

### TEST EXAMPLE 5

### Endothelium-dependent vasodilation activity of the processed ginseng extract of the present invention

Male Sprague-Dawley rats (300-400g) were sacrificed and their thoracic aortae were removed and placed in a modified Krebs-Ringer-bicarbonate solution containing(in mM):NaCl, 118.3; KCl, 4.7; MgSO₄, 1.2; KH₂PO₄, 1.2; CaCl₂, 2.5; NaHCO₃, 25.0; CaEDTA, 0.016; and glucose, 11.1 (control solution). The aortae were cleaned of loose connective tissue and then cut into eight rings (2-3mm wide). In some rings, the endothelium was removed mechanically. The aortic rings were suspended horizontally between two stainless steel stirrups in organ chambers filled with 25ml of control solution (37°C, pH 7.4) and bubbled with 95% O₂ and 5% CO₂. One of the stirrups was anchored to the organ chamber and one was connected to a transducer coupler(Narco bio-system) for the recording of isometric tension. The aortic rings were stretched progressively to the optimal tension(2g) before the addition of phenylephrine(10⁻⁶M). Once the plateau of the contraction to phenylephrine was obtained, the aortic rings were rinsed three times with warm (37°C) control solution. After a resting period (30min), the aortic rings were exposed again to phenylephrine (10⁻⁶M). When the contraction had stabilized, acetylcholine (10⁻⁶M) was added to test the presence or the absence of the endothelium. The organ chambers were rinsed three times with warm (37°C) control solution before the addition of indomethacin (10⁻⁵M) to prevent the production of endogenous vasoactive prostanoids.

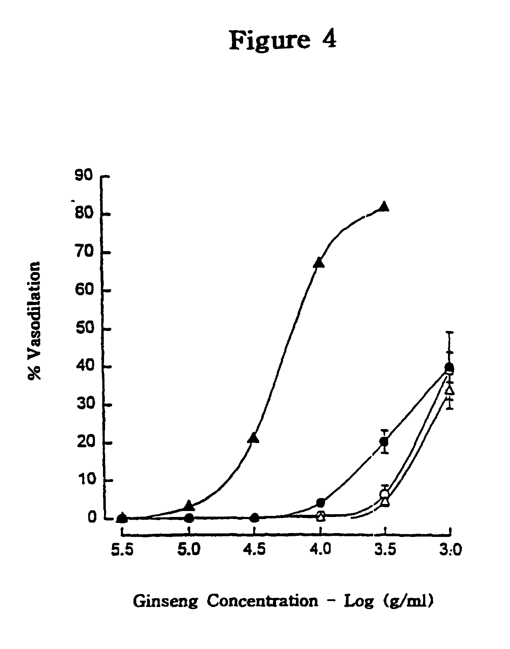

A cumulative concentration-response curve to ginsenoside (10⁻⁶-3x10⁻⁴g/ml) was performed following the concentration of aortic rings with phenylephrine(10⁻⁶M). Ginseng extracts significantly relaxed rat aortic rings contracted with phenylephrine in the presence of the endothelium in a concentration-dependent manner (see, Fig 4). Particularly, the processed ginseng obtained by heat treatment for 2 hours at 120°C according to the present invention shows a vasodilation at much lower concentration. Specifically, the vasodilation activity of the processed ginseng extract having the ratio of (Rg₃+Rg₅)/(Rc+Rd+Rb₁+Rb₂) of above 1, which is produced by heat treatment at 120°C, at 50% contraction level is about 50 times as high as that of fresh ginseng. Such vasodilation activity of the processed ginseng does not show in the blood vessel from which endothelial cells are removed. This means that the vasodilation activity of the processed ginseng is dependent on the endothelial cell.

The result as mentioned above suggests that the processed ginseng of the present invention can be used as an agent for prevention or treatment of disorders resulted from circulatory disturbance such as hypertension, arteriosclerosis, diabetes mellitus, sexual disorder, etc.

### COMPOSITION EXAMPLES

### COMPOSITION EXAMPLE 1 : Beverage composition

| Component | Content(in 100ml) |
|---|---|
| Processed ginseng extract | 360 mg |
| Zingiberis rhizoma extract | 180 mg |
| Ziziphi fructus extract | 1900 mg |
| Cinnamomi cortex extract | 180 mg |
| Lycii fructus extract | 200 mg |
| Taurine | 500 mg |
| Fructose | 10 g |
| Glucose | 0.5g |
| White sugar | 1 g |
| Citric acid | 200 mg |
| Sodium citrate | 100 mg |
| Sodium benzoate | 60 mg |
| Purified water add by | 100 ml |

According to the composition above, fructose, glucose and white sugar were first dissolved in purified water with heating to 95°C, and then cooled slowly to 70°C. Citric acid, sodium citrate and sodium benzoate were dissolved therein while stirring, and subsequently zingiberis rhizoma extract, ziziphi fructus extract, cinnamomi cortex extract, lycii fructus extract and taurine were added thereto while stirring to dissolve. To the resulting solution was added the processed ginseng extract prepared in Example 4 and then the mixture was thoroughly stirred. Sutable amount of purified water was added thereto so that the total volume may be 100ml to prepare 100ml of the beverage composition containing 360mg of the processed ginseng extract.

### COMPOSTTION EXAMPLE 2 : Beverage composition

| Component | Content(in 100ml) |
|---|---|
| Processed ginseng extract | 600 mg |
| Ziziphi fructus extract | 600 mg |
| Polygalae radix extract | 300 mg |
| Cinnamomi cortex extract | 200 mg |
| Taurine | 500 mg |
| Fructose | 10 g |
| Glucose | 0.5g |
| White sugar | 1 g |
| Citric acid | 200 mg |
| Sodium citrate | 100 mg |
| Sodium benzoate | 60 mg |
| Purified water add by | 100 ml |

100ml of the beverage composition containing 600mg of the processed ginseng extract was prepared according to the substantially same method as Composition Example 1 above.

## Claims

1. A processed ginseng or processed ginseng extract **characterized in that** the ratio of ginsenoside (Rg₃+Rg₅)/(Rc+Rd+Rb₁+Rb₂) is above 1.0.

2. The processed ginseng or processed ginseng extract according to claim 1, **characterized in that** it is prepared from ginseng, leaves of ginseng plant or ginseng extract.

3. The processed ginseng or processed ginseng extract according to claim 2, wherein the ginseng is selected from a group consisting of fresh ginseng, white ginseng and fine root of ginseng.

4. A beverage composition comprising the processed ginseng or processed ginseng extract according to any one of claims 1 to 3.

## Patentansprüche

1. Aufbereiteter Ginseng oder aufbereiteter Ginseng-Extrakt, **dadurch gekennzeichnet, dass** das Verhältnis der Ginsenoside (Rg₃+Rg₅)/(Rc+Rd+Rb₁+Rb₂) größer als 1,0 ist.

2. Aufbereiteter Ginseng oder aufbereiteter Ginseng-Extrakt nach Anspruch 1, **dadurch gekennzeichnet, dass** er aus Ginseng, Blättern der Ginseng-Pflanze oder Ginseng-Extrakt hergestellt ist.

3. Aufbereiteter Ginseng oder aufbereiteter Ginseng-Extrakt nach Anspruch 2, **dadurch gekennzeichnet, dass** der Ginseng aus einer Gruppe, bestehend aus frischem Ginseng, weißem Ginseng und feiner GinsengWurzel, ausgewählt ist.

4. Getränks-Zusammensetzung, **dadurch gekennzeichnet, dass** sie den aufbereiteten Ginseng oder den aufbereiteten Ginseng-Extrakt nach einem der Ansprüche 1 bis 3 enthält.

## Revendications

1. Ginseng traité ou extrait de ginseng traité, **caractérisé en ce que** le rapport de ginsenoside (Rg₃+Rg₅)/(Rc+Rd+Rb₁+Rb₂) se situe au-dessus de 1,0.

2. Ginseng traité ou extrait de ginseng traité selon la revendication 1, **caractérisé en ce qu'**il est préparé à partir de ginseng, de feuilles de plante de ginseng ou d'extrait de ginseng.

3. Ginseng traité ou extrait de ginseng traité selon la revendication 2, **caractérisé en ce que** le ginseng est choisi dans le groupe constitué par du ginseng frais, du ginseng blanc et de fines racines de ginseng.

4. Composition de boisson comprenant le ginseng traité ou l'extrait de ginseng traité selon l'une quelconque des revendications 1 à 3.
